Europäisches Patentamt

European Patent Office

Office européen des brevets

(11) Publication number: **0 337 441**
**A2**

# (12) EUROPEAN PATENT APPLICATION

(21) Application number: **89106548.4**

(22) Date of filing: **13.04.89**

(51) Int. Cl.⁴: **C12N 15/00**

(30) Priority: **14.04.88 US 181608**

(43) Date of publication of application:
**18.10.89 Bulletin 89/42**

(84) Designated Contracting States:
**DE FR GB IT**

(71) Applicant: **E.R. Squibb & Sons, Inc.**
**Lawrenceville-Princeton Road**
**Princeton, N.J. 08540(US)**

(72) Inventor: **Haffey, Mary L.**
**16 Fieldstone Place**
**Flemington New Jersey(US)**

(74) Representative: **Vossius & Partner**
**Siebertstrasse 4 P.O. Box 86 07 67**
**D-8000 München 86(DE)**

(54) **Expression of a functional form of a herpes virus DNA polymerase in yeast.**

(57) The herpes simplex virus type 1 (HSV-1) DNA polymerase (pol) gene is expressed in *Saccharomyces cerevesiae*. The gene is cloned into a yeast/*E. coli* shuttle vector fused to the galactokinase gene (GAL-1) promotor.

EP 0 337 441 A2

## EXPRESSION OF A FUNCTIONAL FORM OF A HERPESVIRUS DNA POLYMERASE IN YEAST

The herpes simplex virus (HSV) DNA Polymerase (pol) has been identified as an approximately 140 kiloDalton (kDa) polypeptide which is required for viral DNA replication. The HSV pol is the primary target for antiviral drugs such as acyclovir. In addition, the HSV pol enzyme shares amino acid sequence homology with other herpesvirus DNA polymerases and a variety of eucaryotic DNA polymerases, including the mammalian and yeast alpha polymerases. Thus an improved understanding of the structure of the HSV pol could not only aid in designing new antiviral drugs but could also yield information about the regulation of DNA synthesis, viral replication and cell proliferation. One way in which an improved understanding of the herpesvirus pol structure can be achieved is through the production and purification of functional pol protein. In the past, the only source of pol protein was the infected cell; however, that source has some limitations. While mRNA encoding HSV pol appears to be abundant in the infected cell, only low levels of the pol protein are produced. In addition, several additional polypeptides appear to be tightly associated with the ~140 kDa polypeptide through purification procedures. Therefore, it is difficult to obtain large quantities of pure ~140 kDa protein from the infected cell for study.

Previously, Dorsky et al. (1987. J. Virol. 61(5):1704 - 1707) successfully expressed a functional form of the HSV pol gene in mammalian cells; however, only low levels of active enzyme were produced in that system and the expressed pol enzyme could only be detected by complementation of a temperature sensitive HSV containing a mutation in pol. Dorsky and Crumpacker have also reported (1987. Abstracts of the papers presented at the twelfth International Herpesvirus Workshop. pg. 63) the expression of a '150 kDa' polymerase polypeptide in an *in vitro* translation system, but again, only low levels were produced. In addition, they have also expressed high levels of HSV pol protein in *E. coli*; however, the expressed protein was insoluble and had no readily detectable enzymatic activity.

Previously, McNeil and Friesen (1981. Mol. Gen. Genet.184:386 - 393) had expressed a functional form of another herpesvirus enzyme (thymidine kinase) in yeast and many other foreign genes have been expressed in yeast.

It is, accordingly, on object of the present invention to provide:

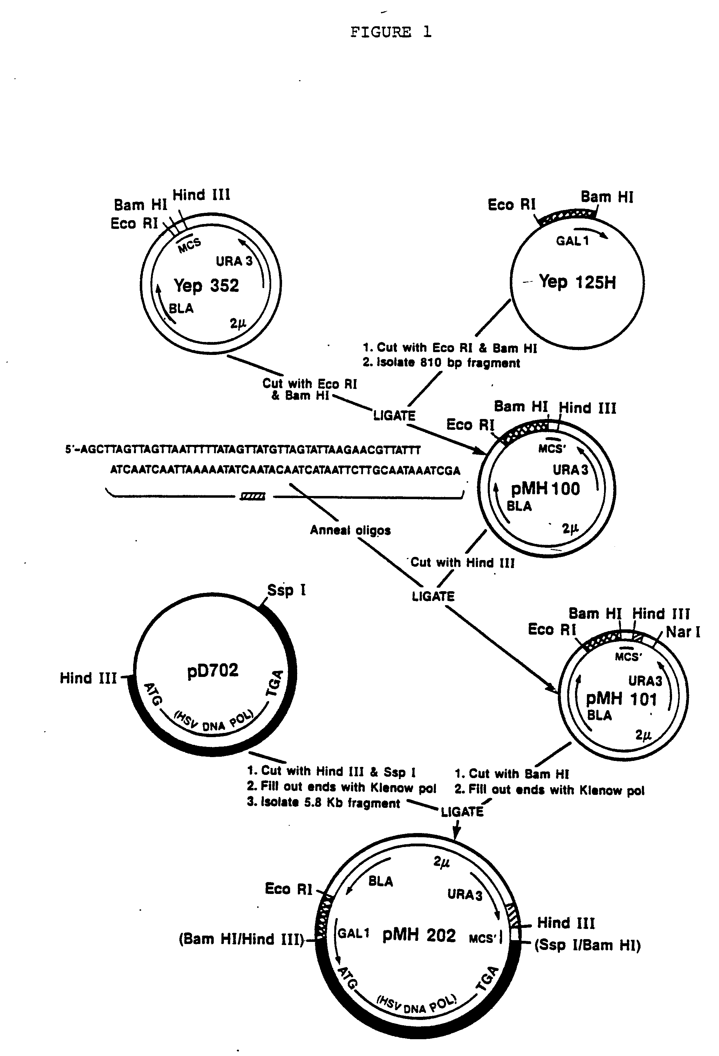

A plasmid expression vector which contains the gene for a herpesvirus DNA polymerase inserted downstream from a promotor as shown in Figure 1 which functions in yeast and inserted in such a manner as to allow for expression of the herpesvirus pol gene.

A plasmid expression vector which contains the gene for a herpes simplex virus DNA polymerase inserted downstream from a promotor as shown in Figure 1 which functions in yeast and inserted in such a manner as to allow for expression of the herpes simplex virus pol gene.

A yeast strain which contains the above described plasmid expression vector containing the gene for herpesvirus DNA polymerase which produces an active form of a herpesvirus DNA polymerase enzyme.

A yeast strain which contains the above described plasmid expression vector containing the gene for herpes simplex virus DNA polymerase which produces an active form of the herpes simplex virus DNA polymerase enzyme.

A process for producing a functional herpesvirus DNA polymerase from a recombinant strain of yeast.

A process for producing a functional herpes simplex virus DNA polymerase from a recombinant strain of yeast.

The above processes involve the following:

(i) constructing an expression vector which contains the herpesvirus pol gene inserted under the control of a promotor which functions in yeast;

(ii) introducing said expression vector into an appropriate yeast strain;

(iii) culturing the resultant recombinant yeast strain under conditions which allow the promotor controlling the herpesvirus pol gene to function and the pol protein to accumulate; and

(iv) lysating the yeast cells to release the herpesvirus pol in a functional form for characterization and purification.

A plasmid expression vector has been constructed which contains a herpesvirus (herpes simplex virus) pol gene under the control of a promotor (galactokinase [GAL-1]; [Johnston, M. and Davis, R. W. 1984. Molecular and Cellular Biology 4(3);1440 - 1448]) which functions in yeast (*Saccharomyces cerevesiae*). That plasmid expression vector was introduced into a yeast strain (Y294 [MAT α; leu2-3, 112; ura 3-52; trp-1; his 3Δ; GAL+; cir+] (Brugge, J. S.; et al. 1987. Molecular and Cellular Biology 7(6):2180 -2187). The resultant, recombinant strain of yeast was grown under conditions (in galactose) which allow for expression of a functional form of the herpesvirus (herpes simplex virus) pol. This strain fulfills the need for a source of directly detectable and functional, HSV pol enzyme distinguishable from other viral proteins.

Figure 1 illustrates the scheme for construction of the expression vector (pMH202) for producing HSV pol in yeast, as described in detail below.

Figure 2 illustrates an immunoblot of the HSV pol protein produced in the yeast cells containing the vector (Y-MH202), as described in detail below.

## Construction of the yeast expression vector pMH202.

The construction of the HSV DNA pol yeast expression vector is summarized in Figure 1. This represents only one example of how such an expression vector can be constructed; other examples may involve the use of:

1. A single copy (centromere) vector; or
2. An integrating vector; or
3. A different promotor can be substituted for the GAL-1 promotor; or
4. A different terminator sequence can be used; or
5. A different herpes simplex virus or herpesvirus pol gene fragment can be used.

The starting plasmid, Yep 352, is a high copy number, *E. coli*/yeast shuttle vector used for the expression of genes which contain a promotor which functions in yeast (HIII, J. E. et al. 1986. Yeast 2:163 - 167). Yep 352 contains the beta-lactamase gene for selective growth in ampicillin containing media in *E. coli* and the URA 3 gene for selective growth in uracil deficient media in yeast. In addition, the vector contains DNA sequences for autonomous replication in bacteria and in yeast. This vector has been modified in order to express foreign genes under the control of a regulatable yeast promotor, the galactokinase promotor (GAL-1). The GAL-1 promotor has been described in detail elsewhere (Johnston, M. and Davies, R. W. above) and has been used successfully by others to express foreign proteins (Wen, D. and Schlesinger, M. J. 1986. PNAS 83: 3639 - 3643). The use of a regulatable promotor enables one to grow cells to high densities before inducing foreign protein expression, thus, avoiding potential deleterious effects of foreign protein accumulation on cell growth. Expression of genes controlled by the gal-1 promotor is induced by galactose, repressed by glucose and uninduced by raffinose.

Unless otherwise noted, all of the recombinant DNA methodologies used in this invention can be found in Maniatis et al. (1982. Molecular Cloning: A Laboratory Manual. Cold Spring Harbor Laboratory. Cold Spring Harbor, N.Y.). The fragment of Yep 352 plasmid DNA used for cloning was obtained by digesting about 5 μg of Yep 352 plasmid DNA with restriction endonucleases (New England Biolabs, Bethesda Research Labs) Eco R1 and Bam HI in order to remove part of the multiple cloning site (MCS) and to generate overhanging 5′ ends. In addition, an approximately 810 base pair (bp) fragment containing the gal 1 - 10 promotor sequence was obtained from plasmid Ycp 125H, a derivative of pBM125 (Johnston, M. and Davis, R. W. above). (Ycp 125H differs from pBM125 only in that it has a HindIII oligonucleotide linker added at the unique BamH1 site. The 810 bp fragment used in this invention is not affected by the addition of the HindIII linker). The approximately 810 bp fragment was obtained by digestion of about 40μg of purified Ycp 125H plasmid DNA with restriction endonucleases: Eco RI and Bam HI. Purification of the DNA fragments was accomplished by running the digested DNAs on a 1% low melting temperature agarose (Bethesda Research Labs) gel and the desired bands were excised with a scapel, transferred to a microfuge tube, heated to 65°C, one half volume of phenol was added at 37°C and the mixture was quickly vortexed and centrifuged at 12,000 rpm in a microfuge. The aqueous phase was removed, re-extracted with one half volume of phenol, the aqueous phase removed again and one tenth volume of 5M NaCl was added to it. The mixture was again centrifuged at 12,000 rpm in a microfuge, the aqueous phase was removed and two volumes of ethanol added to it to precipitate the DNA. The aqueous/ethanol mixture was stored on dry ice for 30 minutes and the DNA pelleted by centrifugation at 12,000 rpm in a microfuge. The DNA pellet was rinsed with 70% ethanol:water, dried under vacuum in a Speed Vac centrifuge and redissolved into a small volume of 10 mM Tris-HCl, 1mM EDTA pH 8.0. The two purified DNA fragments (about 1μg of each) were ligated together at 13°C overnight using T4 DNA ligase (Bethesda Research Labs). About one tenth of that ligation mixture was used to transform frozen competent XL-1 blue *E. coli* (Stratgene Cloning Systems). Recombinant colonies were selected by restriction endonuclease analysis of plasmid preparations from random transformants. The restriction endonucleases used for this analysis were ECOR1 and BamH1 (as above) which release the approximately 810 bp insert from the correctly assembled vector.

In order to improve the expression of foreign genes, a yeast termination sequence was introduced into the vector. The termination sequence was composed of two synthetic oligonucleotides (Figure 1) which anneal to form a fragment containing the yeast iso-1-cytochrome c (CYC-1) gene termination sequence (Zaret, K. S. and Sherman, F. 1982. Cell 28:563 - 573) and translation stop codons in the three possible reading frames. The double stranded oligonucleotide was designed to be directly inserted into the Hind III restriction site in the MCS of pMH100. When inserted in the correct orientation, the oligonucleotide would retain the Hind III site only at the end immediately adjacent to the MCS sequences and upstream of the three stop codons. Two 50 base oligonucleotides containing the cyc-1 termination sequence were synthesized for this study on the Applied Biosystems Model 380B DNA synthesizer using beta-cyanoethyl phosphoramidite chemistry. The oligos were purified by chromatography over a Sepharose G50 medium according to previously described procedures (Applied Biosytems User Bulletin. Issue #13 November 9, 1984). The final concentration of the oligonucleotides was about 7 - 8 $\mu$g/ml in 10 mM Tris-HCl pH 8.0, 1mM EDTA. Approximately 15 $\mu$g of each purified oligo were placed in a microfuge tube and annealed by heating the mixture to 55°C for 5 minutes and allowing it to cool at room temperature for 30 minutes. In addition, about 2$\mu$g of purified, linear pMH100 was prepared by digestion of the plasmid DNA with restriction endonuclease Hind III to create a linear molecule and gel purification of the linear DNA from a low melting temperature agarose gel was accomplished as described above. About 2$\mu$g of the above linear plasmid and 30$\mu$g of the oligo DNAS were ligated overnight at 13°C using T4 DNA ligase (as above). About one tenth of the ligation mixture was used to transform frozen competent HB101 E. coli (Bethesda Research Labs). Recombinant colonies were chosen at random and screened by restriction endonuclease analysis of plasmid preparations from random transformants and the proper orientation of the insert was determined by specific differences (compared to pMH100) in the patterns generated by digestion of recombinant plasmids with appropriate restriction enzymes. The restriction endonucleases used for this analysis were EcoR1 and Nar1 and in a separate assay Nar1 and Hind III. In both cases, the correct new construction would yield a short DNA fragment which differs from the fragment generated in the starting vector, pMH100, by the addition of 50 bp. The new construction containing the correctly oriented 50 bp oligonucleotide was designated pMH101.

pMH101 was digested with restriction endonuclease Bam H1 (as above) and the overhanging 5' ends were filled out with E. coli DNA polymerase (Klenow fragment) (Bethesda Research Labs) as described in Maniatis et al. (above) to create blunt ends which could accept a DNA fragment containing the HSV pol gene. The HSV pol gene was isolated form plasmid pD702. Plasmid pD702 (Dorsky and Crumpacker above) was digested with restriction enzymes Hind III and SspI and the overhanging 5' ends made blunt, as described above. The 5.8 kilobase pair (kb) Hind III-SspI fragment containing the entire HSV-1 pol gene and the linear, blunt ended pMH101 were both gel purified from low melting temperature agarose as described above. About 2 $\mu$g of vector and 1 $\mu$g of pol insert DNA were then ligated together at 13°C overnight (as above). About one tenth of the ligation mixture was used to transform frozen competent XL-1 Blue E. coli - (as above). Recombinant isolates were selected by colony hybridization as described in Maniatis et al. (above) using a probe consisting of the 5.8 kt fragment which had been radiolabeled with $\alpha$-$^{32}$P-dCTP (3000 Ci/mmol Amersham) using a commercially available nick translation kit (Bethesda Research Labs). The proper orientation of the inserted gene was determined by digestion with an appropriate restriction endonuclease. The restriction enzyme used was EcoR1 which cuts only once in the vector. The final construction was designated pMH202.

Under inducing conditions in yeast, the final construction (pMH202) was designed to generate a transcript which contains ~140 bases of 5' untranslated sequence before the natural ATG initiation codon of the HSV pol gene and about 2.1 kilobases downstream of the natural UGA termination codon before reaching the transcription termination oligonucleotide sequence. Therefore, the vector should encode an RNA which can be translated to the complete HSV DNA polymerase polypeptide as predicted from the DNA sequence (Quinn, J.P. and McGeoch, D. J. 1985. Nucleic Acids Res. 13:8143 - 8163).

## Expression ofHSV DNA Polymerase in *Saccharomyces cerevesiae*

In order to determine whether HSV pol protein was expressed and whether HSV specific DNA polymerase activity could be detected in yeast cells containing pMH202, the following experiments were performed. This description represents only one example of how such a strain can be constructed. Other examples may include a different yeast host strain and/or a different transformation procedure. Yeast cells (Y 294) were transformed with pMH101 (control plasmid without the HSV pol gene) or pMH202 (expression plasmid with HSV pol gene) DNAs. Transformation of the yeast cells was accomplished using the method of J. D. Beggs (1978. Nature 275: 104 - 109). The transformants were selected for uracil prototrophy by growth on minimal media containing 182.2 g/L sorbitol; 20 g/L glucose; 6.7 g/L yeast nitrogen base (Difco) supplemented with 30μg/ml leucine, 20μg/ml histidine, 20μg/ml tryptophan and 2% glucose but lacking uracil. One colony of each type (designated Y-MH101 or Y-MH202, respectively) was selected at random for further analysis. 100 ml overnight cultures were grown from these two strains in minimal media (0.7% yeast nitrogen base [Difco]) containing the required amino acids as described above and 2% raffinose. The following day, cultures were divided in half, cells were pelleted, resuspended in 100 ml of fresh media containing either 2% raffinose or 2% galactose and again grown overnight. The following day, the four cultures were pelleted at 4°C. Cell lysates were prepared as described by Johnson et al. (1985 . Cell 43:369 - 377). This represents only one example of how such a lysate may be prepared. Cell pellets were resuspended in 1 ml 50 mM Tris-HCl, pH 8.0; 50mM NaCl, 1% dimethylsulfoxide; 10% glycerol; 5mM beta-mercaptoethanol; 10mM EDTA; 2mM benzamidine; and 1mM phenylmethylsulfonylfluoride (PMSF). Lysis was achieved by vortexing the cells 15 - 20 times for 1 minute in the presence of an equal volume of acid-washed glass beads. The extent of lysis was monitored by microscopy. The lysed extracts were transferred to 1.5 ml microfuge tubes and centrifuged for 10 minutes at 12,000 x g. Extracts were stored at -80°C in 15% glycerol until use. The final protein concentration of each of the extracts was between 5 - 6 mg/ml.

Infected cell extracts were prepared essentially as described by Karkas et al. (1986. J. Med. Chem. 29:842 - 848). HeLa S3 cells were infected with HSV-1 KOS at a multiplicity of infection of 10 plaque forming units per cell and harvested at 16 hours post infection. Cell pellets (~1 x $10^8$ cells) were frozen overnight at -80°C; thawed on ice; resuspended in 20 ml of hypotonic solution A: (10mM $K_2 PO_4$, pH 7.0; 10mM KCl; 1mM PMSF; 1mM dithiothreitol [DTT]) and incubated on ice for 30 minutes with occasional vortexing. An equal volume of solution B:(0.7M $K_2 PO_4$, pH 7.0; 28% glycerol; 6mM DTT; 0.4% NP-40; 1mM PMSF) was added and the mixture incubated on ice for an additional 30 minutes with occasional vortexing. The mixture was centrifuged for 1 hour at 125,000 x g and NP-40 added to a final concentration of 0.4%. Aliquots were stored frozen at -80°C. The final protein concentration of the extract was 5 - 6 mg/ml.

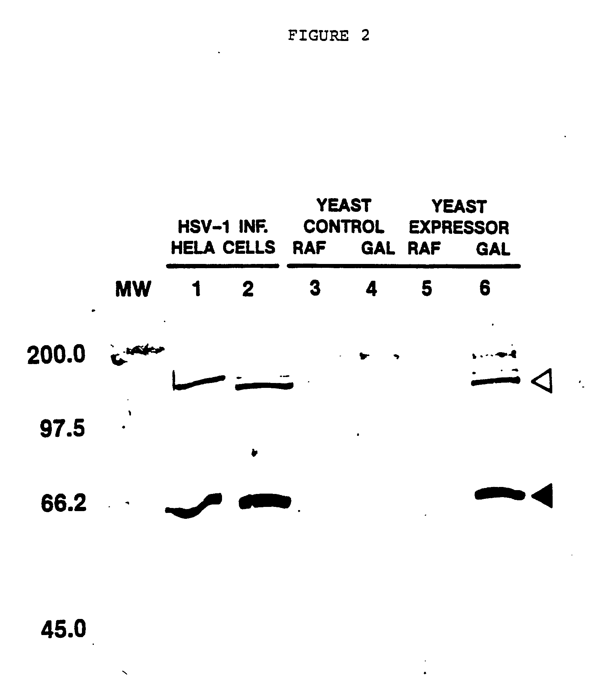

The recombinant cultures were screened for the production of the HSV DNA polymerase polypeptide by analysis of the enzyme extract lysates described above on a 10% polyacrylamide gel according to the procedure of Heine et al. (1974, J. Virol. 14:640 - 651). After the lysates were run on SDS-PAGE, they were transferred to nitrocellulose as described by Towbin et al. (1979, PNAS 76:4350 - 4354) and the processing of filters was performed using an Immun-Blot assay kit from BioRad, except that bovine serum albumin (BSA) was substituted for gelatin. Immunoblotting was performed using an antiserum which was prepared against a highly purified HSV-1 DNA polymerase preparation. The antiserum was raised in a New Zealand White Rabbit by a series of five immunizations using 10μg of purified polymerase per dose, the first injection using complete, and the subsequent injections using incomplete Freund's adjuvant. A 1:200 dilution of the primary rabbit antibody and a 1:1000 dilution of the anti-rabbit alkaline phosphatase conjugate was used. The results of the immunoblot procedure are shown in Figure 2. For comparison, lane 1 contains about 9μg of the HSV-1 DNA pol from the highly purified preparation used as immunogen to prepare our antiserum and lane 2 contains about 60μg crude HSV-1 pol preparation from infected HeLa cells prepared as a control for the pol enzyme assays. Lanes 3 - 6 (about 50 - 60 μg/lane) contain the yeast cell extracts as indicated. The data clearly show the specific induction of an immunoreactive polypeptide of approximately 140 kDa only in Y-MH202 cells grown under inducing condition (lane 6). In addition, the yeast expressed polypeptide exhibits the same electrophoretic mobility as the HSV-1 pol from crude infected cell extracts. It should be noted that the pol polypeptide in lane 1 (derived from strain HSV-1 mP) has a slightly slower electrophoretic mobility than the predominant high molecular weight pol protein band in lane 2 (derived from strain HSV-1 KOS). In addition, it should be noted that our antiserum also reacts with a HSV specific 65 kDa protein found in infected cells and in the purified DNA pol immunogen. In the yeast cell extracts this 65 kDa protein represents a degradation product of the 140 kDa HSV DNA pol polypeptide. However, the 65 kDa protein in lanes 1 and 2 may also contain the 65 kDa HSV DNA binding protein which is often found tightly associated with 140 kDa DNA pol polypeptide through purification procedures. The antiserum of this invention has been shown to react with this 65 kDa DNA binding protein by immunoprecipitation.

The lysates were also analyzed for DNA polymerase enzyme activity. Each of the four yeast cell extracts was assayed for DNA pol activity under two sets of conditions. The yeast DNA pol assay conditions were designed to detect the alpha polymerase which should be present in all lysates. The HSV DNA pol assay conditions were chosen to detect HSV specific DNA pol activity which is enhanced by high salt (100mM ammonium sulfate). In contrast, the yeast alpha DNA pol is inhibited under these salt conditions. The assay for yeast DNA polymerase was performed essentially as described in Celniker and Campbell (1982. Cell 31:201 - 213), but without addition of ribonucleotide triphosphates. Reaction mixtures contained 5μl of enzyme extract (25 - 30 μg of protein) in 10mM Tris-HCl, pH 7.5; 10 mM MgCl₂; 10 mM (NH₄)₂SO₄; 0.1 mM DTT; 100 μM each of dATP, dCTP, dGTP; 100 μM ³H-TTP (10 cpm/pmol); 40 μg/ml nicked calf thymus DNA; and 2 mM spermidine in a final volume of 50 μl. The assay for the HSV-1 DNA pol was performed as follows. The reaction mixture contained 5 μl of enzyme extract (25 pg of protein) 50mM Tris-HCl, pH 8.0; 5mM MgCl; 100 mM (NH₄)₂SO₄; 1 mM DTT; 5μM dATP, dCTP, dGTP; 5 μM ³H-TTP (200 cpm pmol); 30 μg/ml nicked calf thymus DNA; and 100 μg/ml BSA in a final volume of 50 μl. All DNA polymerase assays were performed by incubating the reactants for 15 minutes at 37°C; the reactions were spotted onto glass fiber filters and quenched by the addition of 10 mM sodium pyrophosphate in 5% TCA. The filters were washed thoroughly, twice using 1N HCl and once using 95% ethanol; air dried and quantitated by scintillation counting using a LKB 1214 Rackbeta counter at 63% efficiency. All assays were conducted in duplicate.

Table 1 reveals that only Y-MH202, HSV DNA pol expressor cells, grown under inducing conditions (galactose) produce significant amounts of high salt DNA pol activity. In addition, Table 2 reveals that this novel high salt activity in the Y-MH202 yeast cell extracts is inhibited by 23 μM acyclovir triphosphate (ACVTP) and 5.3 μM aphidicolin, both well known inhibitors of the HSV DNA pol enzyme. Table 2 also reveals that the low salt DNA pol activity in all of the extracts tested is sensitive aphidicolin (which is characteristic of the alpha polymerases) and is not sensitive to ACVTP at the concentration tested.

These results indicate the successful expression of an active form of the HSV DNA pol in galactose-induced Y-MH202 yeast cells.

The expression of an active form of the HSV DNA pol in yeast should facilitate studies on the structure of the enzyme. The yeast expressed HSV DNA polymerase enzyme can be characterized with respect to substrate specificity and inhibition kinetics. Site-specific mutations can be be engineered into the gene in order to evaluate their effects on enzyme activity and intracellular localization. Purification of the protein should enable physical/chemical studies on the three dimensional structure and functional domains of the protein, i.e., substrate/inhibitor binding sites. In addition, it may be possible to express other components of the HSV origin specific DNA replication complex, as described by Challberg (1986. PNAS 83:9094 - 9098), in *Saccharomyces* and evaluate their interaction with the 140 kDa DNA polymerase protein and their effect on DNA polymerase activity.

Table 1.  Induction of DNA Polymerase in Yeast

| | HSV DNA Polymerase Activity[a] | | | |
|---|---|---|---|---|
| | Yeast Control Y-MH101 | | Yeast Expressor Y-MH202 | |
| Growth Conditions | | | | |
| | Expt 1 | Expt 2 | Expt 1 | Expt 2 |
| Uninduced (Raffinose) | 52 | 36 | 0[b] | 80 |
| Induced (Galactose) | 22 | 87 | 1337 | 1106 |

| | Yeast Alpha Pol Activity[a] | | | |
|---|---|---|---|---|
| | Yeast Control Y-MH101 | | Yeast Expressor Y-MH202 | |
| Growth Conditions | | | | |
| | Expt 1 | Expt 2 | Expt 1 | Expt 2 |
| Uninduced (Raffinose) | 1396 | 1132 | 1291 | 1396 |
| Induced (Galactose) | 956 | 1254 | 1324 | 1296 |

a.  Pol activity expressed as cpm of $^3$H-TTP incorporated per reaction (background subtracted); assay conditions as described above.

b.  "0" indicates value below background.

Table 2.  Inhibition of DNA POL Activity by Acyclovir
Triphosphate and Aphidicolin

| Extract [a] | Assay [b] Conditions | % Inhibition by Drug [c] | |
|---|---|---|---|
| | | ACVTP | Aphidicolin |
| Yeast Control | HSV pol | - | - |
| (galactose) | alpha pol | 0 | 88 |
| Yeast Expressor | HSV pol | 93 | 94 |
| (galactose) | alpha pol | 0 | 80 |
| HSV-infected | HSV pol | 89 | 90 |
| HeLa cells | alpha pol | 0 | 89 |

a.  Extracts were prepared as described above.  Yeast
control (Y-MH101) and expressor (Y-MH202) cell extracts
were prepared from cultures grown in the presence of
galactose.

b.  Assay conditions were as described above.

c.  Acyclovir triphosphate (ACVTP) and aphidicolin were
present in reactions at 23 µM and 5.3 µM, respectfully.
% inhibition is determined by subtracting the relative
activity (ratio of cpm incorporated per reaction with
drug/reaction without drug) from 1.0 and multiplying by
100%.  0% values indicate relative activity was ≧1.0.
(-) indicates value is not statistically significant
due to low pol activity.

**Claims**

1. A plasmid expression vector which contains the gene for a herpesvirus DNA polymerase inserted downstream from a promotor which functions in yeast and inserted in such a manner as to allow for expression of the herpesvirus pol gene.

2. The plasmid expression vector according to claim 1, wherein the gene encodes a herpes simplex virus DNA polymerase.

3. A yeast strain which contains the plasmid expression vector according to claim 1, which produces an active form of the herpesvirus DNA polymerase enzyme.

4. A yeast strain which contains the plasmid expression vector of claim 2 which produces an active form of the herpes simplex virus DNA polymerase enzyme.

5. A process for producing a functional herpesvirus DNA polymerase from a recombinant strain of yeast which comprises

(i) constructing an expression vector which contains the herpesvirus pol gene inserted under the control of a promotor which functions in yeast;

(ii) introducing said expression vector into an appropriate yeast strain;

(iii) culturing the resultant recombinant yeast strain under conditions which allow the promotor controlling the herpesvirus pol gene to function and the pol protein to accumulate; and

(iv) lysating the yeast cells to release the herpesvirus pol in a functional form for characterization and purification.

6. A process according to claim 5 wherein the herpesvirus DNA polymerase is a herpes simplex virus DNA polymerase.

## FIGURE 1

5'-AGCTTAGTTAGTTAATTTTTATAGTTATGTTAGTATTAAGAACGTTATTT
   ATCAATCAATTAAAAATATCAATACAATCATAATTCTTGCAATAAATCGA

FIGURE 2